# EUROPEAN PATENT APPLICATION

(11) **EP 4 129 227 A1**
(43) Date of publication of application: **08.02.2023**
(21) Application number: 21775903.4
(22) Date of filing: 16.03.2021
(51) Int. Cl.: A61B 34/30

(54) **SURGICAL INSTRUMENT, SURGICAL INSTRUMENT SYSTEM, AND SURGICAL ROBOT**

(30) Priority: 24.03.2020 CN 202010213900
(71) Applicant: Shanghai Microport Medbot (Group) Co., Ltd., Shanghai 201203 (CN)
(72) Inventor: JIANG, Youkun, Shanghai 201203 (CN); HE, Chao, Shanghai 201203 (CN); HE, Yuyuan, Shanghai 201203 (CN); CHANG, Xinchao, Shanghai 201203 (CN)
(74) Representative: Patentship Patentanwaltsgesellschaft mbH
(86) International application number: PCT/CN2021/081183
(87) International publication number: WO 2021/190365

(57) **Abstract**

A surgical instrument, a surgical instrument system and a surgical robot. The surgical instrument includes an end portion (100), the end portion (100) including at least one joint defined with a zero position and a non-zero position; the surgical instrument further comprising a joint drive shaft (2000) and a reset assembly, the joint drive shaft (2000) connected with the joint of the end portion in a force transmitable manner; the reset assembly including a reset shaft (3100), a transmission mechanism (3200) and a reset mechanism (3300), and the reset shaft (3100) connected with the joint drive shaft (2000) by a transmission mechanism (3200), and the transmission mechanism (3200) being a reduction transmission mechanism; when the joint of the end is in the non-zero position, the reset mechanism (3300) is configured to drive the reset shaft (3100) to rotate, and then drive the joint drive shaft (200) to rotate through the transmission mechanism (3200), so as to drive the joint of the end portion to move to the zero position. The invention utilizes the reset assembly to adjust the joint of the end portion (100) to reset from the non-zero position to the zero position without causing contamination to the surgical instrument.

## Description

### TECHNICAL FIELD

The present invention relates to the technical field of medical instruments, in particular to a surgical instrument, a surgical instrument system and a surgical robot.

### BACKGROUND

When using a surgical instrument, especially a robotic end multi-joint surgical instrument for minimally invasive surgery, the operator needs to touch the end portion of the surgical instrument by hand to adjust the end portion to the zero position before the end portion is inserted into the trocar. The so-called zero position is the definition of a state of the surgical instrument, such as the state in which each joint of the end portion of the surgical instrument and the instrument shaft are on the same axis, and other states correspond to non-zero positions. Adjusting the end portion of the surgical instrument to be at the zero position can facilitate the insertion of the end portion into the trocar. However, the operator directly adjusts the end portion of the surgical instrument by hand, which is likely to cause contamination to the end portion of the surgical instrument, and after the use of the end portion of the surgical instrument is complete during the surgical operation, the end portion of the surgical instrument is still located in the human body and is in a non-zero position, and it is difficult for its joints to return to the state where each of them is coaxial with the corresponding instrument shaft (that is, it is difficult for the end portion of the surgical instrument to return to the zero position). As a result, the end portion of the surgical instrument will collide with the edge of the trocar when the end portion of the surgical instrument is pulled out from the trocar, resulting in damage to the end portion of the surgical instrument.

### SUMMARY OF THE INVENTION

An object of the present invention is to provide a surgical instrument, a surgical instrument system and a surgical robot, so as to solve the problem that the end portion of the existing surgical instrument is difficult to smoothly insert into and pull out from the trocar when the end portion is in a non-zero position, and also to avoid the problem of contamination of the end portion of the surgical instrument caused by the operator directly touching the end portion.

In order to achieve the above object, the present invention provides a surgical instrument, comprising an end portion and an instrument operating device, the end portion comprising at least one joint defined with a zero position and a non-zero position; the instrument operating device comprising a joint drive shaft and a reset assembly, and the joint drive shaft connected with the joint of the end portion in a force transmitable manner; the reset assembly comprising a reset shaft, a transmission mechanism and a reset mechanism, and the reset shaft connected with the joint drive shaft by the transmission mechanism, and wherein
when the joint of the end portion is in the non-zero position, the reset mechanism is configured to drive the reset shaft to rotate, to drive the joint drive shaft to rotate through the transmission mechanism, so as to drive the corresponding joint of the end portion to move to the zero position.

Optionally, the instrument operating device comprises a first basal plate and a second basal plate that are arranged opposite to each other, the first basal plate and the second basal plate spaced apart from each other and remaining stationary relative to each other, the joint drive shaft rotatably disposed on the first basal plate and extending through the second basal plate, the reset shaft rotatably disposed on the second basal plate, and connected with a portion of the joint drive shaft extending out of the second basal plate by the transmission mechanism.

Optionally, a reduction ratio of the transmission mechanism ranges from 1: 1 to 5:1.

Optionally, the transmission mechanism is an engagement transmission mechanism.

Optionally, the transmission mechanism is a gear transmission mechanism comprising a driving gear and a driven gear, the driving gear configured to rotate synchronously with the reset shaft, and the driven gear configured to rotate synchronously with the joint drive shaft, and the driven gear engaged with the driving gear.

Optionally, the transmission mechanism is a flat belt transmission mechanism comprising a driving pulley, a driven pulley and a transmission belt, the driving pulley configured to rotate synchronously with the reset shaft, the driven pulley configured to rotate synchronously with the joint drive shaft, and the transmission belt is wound around the driving pulley and the driven pulley.

Optionally, the reset mechanism comprises a drive member, a force transmission member and an elastic support, and the force transmission member disposed on the reset shaft and remaining stationary relative to the reset shaft, the drive member configured to drive the reset shaft to rotate through the force transmission member, and the elastic support configured to allow and disallow a force transmission between the reset shaft and the drive member.

Optionally, the drive member is sleeved on the reset shaft, and remains stationary in a circumferential direction thereof relative to the second basal plate, and an end face of the drive member facing the force transmission member is formed as a guide surface; wherein
the drive member, the force transmission member and the elastic support are configured such that when the elastic support is not subjected to an external force causing a deformation thereof, the elastic support is configured to support the drive member so that the drive member is apart from the force transmission member; when the joint of the end portion is in the non-zero position and the elastic support is compressed under an action of an external force, the drive member moves toward the force transmission member to cause the guide surface to guide the force transmission member to rotate around an axis of the reset shaft so that the reset shaft is drived to rotate to move the corresponding joint of the end portion to the zero position.

Optionally, when the drive member is in contact with the force transmission member, the force transmission member is movable on the guide surface, and the guide surface provides a force for rotating the force transmission member to drive the force transmission member to rotate around the axis of the reset shaft.

Optionally, the surgical instrument further comprises a limit mechanism configured to prevent the reset shaft from rotating when the corresponding joint of the end portion moves to the the zero position.

Optionally, the limiting mechanism is a zero position slot defined in the guide surface, and the zero position slot is circumferentially sized to match with force transmission member; when the joint of the end portion returns to the zero position, the force transmission member is accommodated in the zero position slot.

Optionally, wherein the guide surface has a peak and a valley, and the zero position slot is arranged at the valley.

Optionally, the drive member comprises a first magnet, and the force transmission member comprises a second magnet, and wherein the first magnet is connected to the elastic support, and the first magnet is sleeved on the reset shaft and remains stationary in a circumferential direction thereof relative to the second basal plate, and the first magnet is movable along an axial direction of the reset shaft, and the second magnet is fixedly arranged on the reset shaft; wherein
the drive member and the elastic support are configured such that when the elastic support is not subjected to an external force causing a deformation thereof, no magnetic force is generated between the first magnet and the second magnet; when the joint of the end portion is in the non-zero position and the elastic support is compressed and deformed under an action of an external force, the first magnet moves toward the second magnet, so that a magnetic force is generated between the first magnet and the second magnet to drive the reset shaft to rotate for causing the corresponding joint of the end portion to move to the zero position.

Optionally, the first magnet has a first surface and the second magnet has a second surface, and the first surface faces the second surface, and wherein at least one N pole and at least one S pole are arranged on the first surface, and at least one N pole and at least one S pole are arranged on the second surface, when the joint of the end portion is in the zero position, the N pole on the first surface is arranged directly opposite to the S pole on the second surface, and the S pole on the first surface is arranged directly opposite to the N pole on the second surface.

Optionally, the reset mechanism further comprises a pressing portion configured to allow the drive member to be connected to the elastic support, and keep the drive member stationary in a circumferential direction thereof relative to the second basal plate.

Optionally, a number of the elastic support is one, a number of the reset shafts is at least two, and the at least two drive shafts are evenly arranged around the one elastic support.

Optionally, a blind mounting hole is formed on the drive member and sleeved on the reset shaft, and the elastic support is disposed in the blind mounting hole; the elastic support has a first end and a second end that are opposite to each other along an axial direction of the reset shaft, the first end abutting on a top wall of the blind mounting hole, and the second end abutting against an end face of the reset shaft.

Optionally, a blind mounting hole is formed on the drive member, a bearing is provided at the blind mounting hole, and the reset shaft is configured to be inserted into the blind mounting hole and connected with the drive member by the bearing; the elastic support is arranged in the blind mounting hole, and the elastic support has a first end and a second end that are opposite to each other along an axial direction of the reset shaft, the first end abutting on a top wall of the blind mounting hole, and the second end fixedly connected with the bearing.

Optionally, the reset mechanism further comprises a seat defined therein with a through hole, the seat perpendicular to the reset shaft and arranged at a side of the second basal plate away from the first basal plate, the seat remaining stationary relative to the second basal plate, and the drive member configured to move in the through hole along the axial direction of the rest shaft.

Optionally, a protrusion is provided at the through hole, the drive member is defined thereon with a groove extending along an axial direction of the drive member, and the protrusion is matched with the groove, so that the drive member remains stationary in the circumferential direction relative to the second basal plate.

Optionally, a zero position slot is formed in the guide surface, and the zero position slot is circumferentially sized to match with the force transmission member; when the joint of the end portion returns to the zero position, the force transmission member is accommodated in the zero position slot, and the zero position slot is aligned with the groove in the circumferential direction of the drive member.

Optionally, the reset mechanism comprises a torsion spring that is sleeved on the reset shaft and has a third end and a fourth end that are opposite to each other, the third end configured to remain stationary in a circumferential direction thereof relative to the second basal plate, the fourth end connected with the reset shaft, and the fourth end being rotatable synchronously with the reset shaft;
the torsion spring is configured to store elastic potential energy when the joint of the end portion is in the non-zero position, and to drive the reset shaft to rotate and drive the joint drive shaft to rotate when the torsion spring releases the elastic potential energy, so as to move the joint of the end portion to the zero position.

Optionally, the reset mechanism comprises a connection plate connected with the third end of the torsion spring, so that the third end remains stationary in a circumferential direction thereof relative to the second basal plate.

In order to achieve the above object, the present invention provides a surgical instrument system, comprising a power case and the above surgical instrument, wherein the joint drive shaft is configured to receive power output from the power case and drive the joint to move.

In order to achieve the above object, the present invention provides a surgical robot, comprising the above surgical instrument system and a robotic arm configured to connect with the surgical instrument system.

Compared with the prior art, the surgical instrument, the surgical instrument system and the surgical robot of the present invention have the following advantages:
The aforementioned surgical instrument includes an end portion. The end portion includes at least one joint defined with a zero position and a non-zero position. The surgical instrument further includes a joint drive shaft and a reset assembly. The joint drive shaft is connected with the joint of the end portion in a force transmitable manner. The reset assembly includes a reset shaft, a transmission mechanism and a reset mechanism. The reset shaft is connected with the joint drive shaft by the transmission mechanism. The transmission mechanism is a deceleration transmission mechanism. When the joint of the end portion is in the non-zero position, the reset mechanism is configured to drive the reset shaft to rotate, and then drive the joint drive shaft to rotate through the transmission mechanism, so as to drive the joint of the end portion to moved to the zero position. By driving the joint of the end portion to return to the zero position through the reset assembly, it prevents the end portion from being directly touched by the operator during the adjustement of the posture of the end portion, and reduces the risk of contamination of the end portion. After the use of the surgical instrument is complete during the surgical operation, the joint of the end portion can be easily adjusted to move to the zero position. As a result, when the end portion is pulled out of the trocar, the transmission mechanism can be prevented from being damaged due to collision with the trocar. For a surgical instrument whose unidirectional rotation angle is greater than the unidirectional rotation angle of the reset shaft, by adjusting the transmission ratio of the transmission mechanism, the joint of the end portion can be reset to the zero position from a non-zero position deviating from the zero position at any angle.

### BRIEF DESCRIPTION OF THE DRAWINGS

Fig. 1 is a schematic diagram showing a partial structure of a surgical instrument according to a first embodiment of the present invention;
Fig. 2 is a schematic structural diagram of the end portion of the surgical instrument according to the first embodiment of the present invention, in which the end portion is in a non-zero position;
Fig. 3 is a schematic diagram showing a partial structure of a surgical instrument according to the first embodiment of the present invention, in which the elastic support is compressed;
Fig. 4 is a schematic structural diagram of the end portion of the surgical instrument according to the first embodiment of the present invention, in which the end portion is in the zero position;
Fig. 5 is a schematic diagram showing a partial structure of a surgical instrument according to the first embodiment of the present invention, in which one reset shaft, one joint drive shaft and one transmission mechanism are shown;
Fig. 6 is a schematic diagram showing a partial structure of a surgical instrument according to the first embodiment of the present invention, in which the reset shaft is provided with a drive member, and the reset shaft is connected to one joint drive shaft by one transmission mechanism;
Fig. 7 is a schematic diagram showing a partial structure of a surgical instrument according to a second embodiment of the present invention;
Fig. 8 is a schematic diagram showing a partial structure of a surgical instrument according to a third embodiment of the present invention;
Fig. 9 is a partial cross-sectional view of the surgical instrument shown in Fig. 8;
Fig. 10 is a schematic diagram showing a partial structure of a surgical instrument according to a fourth embodiment of the present invention;
Fig. 11 is a schematic diagram showing a partial structure of a surgical instrument according to the fourth embodiment of the present invention, in which one reset shaft is shown;
Fig. 12 is a schematic diagram of a drive member of a surgical instrument according to the fourth embodiment of the present invention;
Fig. 13 is a schematic diagram showing a partial structure of a surgical instrument according to a fifth embodiment of the present invention;
Fig. 14 is a schematic structural diagram of a surgical instrument system according to an embodiment of the present invention;
Fig. 15 is a schematic diagram showing a surgical robot is in operation according to an embodiment of the present invention.

List of Reference Numerals:
100: end portion;
110: end base; 120: actuator base; 130: end actuator;
200: case portion;
1000: base;
1100: first basal plate; 1200: second basal plate;
2000: joint drive shaft;
2000a: first joint drive shaft; 2000b: second joint drive shaft; 2000c: third joint drive shaft; 2000d: fourth joint drive shaft;
3100: reset shaft;
3200: transmission mechanism;
3210: driving gear; 3220: driven gear; 3230: driving pulley; 3240: driven pulley; 3250: transmission belt;
3300: reset mechanism;
3310: drive member;
3311: guide surface; 3312: zero position slot, 3313: groove;
3320: force transmission member;
3330: elastic support;
3340: pressing portion;
3341: connection plate, 3342: button;
3350: seat;
3351: through hole;
3360: torsion spring;
300: instrument shaft;
10: power case;
20: sterile accessory;
30: trocar;
1: console; 2: surgical cart; 3: side cart;
L1: first axis; L2: second axis; L3: third axis.

### DETAILED DESCRIPTION

The core idea of the present invention is to provide a surgical instrument including an end portion and a case portion, the end portion includes at least one joint defined with a zero position and a non-zero position. The case portion includes a joint drive shaft and a reset assembly, and the joint drive shaft is connected with the joint of the end portion in a force transmittable manner. The reset assembly includes a reset shaft, a transmission mechanism and a reset mechanism, and the reset shaft is connected with the joint drive shaft through the transmission mechanism. When the joint of the end portion is in the non-zero position, the reset mechanism is configured to drive the reset shaft to rotate, and then drive the joint drive shaft to rotate by means of the transmission mechanism, so as to drive the joint of the end portion to move to the zero position.

In other words, in embodiments of the present invention, when the joint at the end portion of the instrument is in the non-zero position, the reset assembly is configured to adjust the joint of the end portion, so that the joint of the end portion returns to the zero position, during which the operator does not need to directly touch the end portion for adjustment. In an aspect, contamination of the end portion of the instrument due to direct contact with the operator's hand is avoided. In another aspect, when the end portion of the surgical instrument is in the human body and in a non-zero position, the operator can adjust the end portion ouside the human body (i.e., at the proximal end of the surgical instrument) so that the joint of the end portion returns to the zero position. As a result, the end portion of the surgical instrument can be smoothly pulled out of the trocar.

In particular, when the transmission mechanism is a deceleration transmission mechanism, and the unidirectional rotation angle of the joint of the end portion is greater than the unidirectional rotation angle of the reset shaft, an appropriate transmission ratio set for the transmission mechanism allows the joint of the end portion to be moved from a non-zero position at any angle to the zero position.

To make the objectives, advantages and features of the present invention clearer, t the present invention will be further described in detail below with reference to the figures. It is noted that the figures are provided in a very simplified form not necessarily presented to scale, with their only intention to facilitate convenience and clarity in explaining the disclosed embodiments.

As used herein and in the appended claims, the singular forms of "a", "an", and "the", include plural references unless the context clearly dictates otherwise. As used in this specification, the term "or" is generally employed in its sense including "and/or" unless the content clearly dictates otherwise, and the terms "installed", "connected", "coupled" shall be understood in a broad sense, for example, it may be a fixed connection, a detachable connection, or an integral connection. It can be a mechanical connection or an electrical connection. It can be directly connected, or indirectly connected through an intermediate medium, and it can be the internal communication between two elements or the interaction relationship between the two elements. For those of ordinary skill in the art, the specific meanings of the above terms in the present invention can be understood according to specific situations. Throughout the figures, like reference numerals indicate the same or analogous components or elements.

The orientations or positional relationships indicated by "up", "down", "left", "right", "clockwise", "counterclockwise", etc. mentioned herein are all based on the orientations or positional relationships shown in the accompanying drawings. It is only for the convenience of describing the embodiments of the present invention, rather than indicating or implying that the indicated devices or elements must have a specific orientation and be configured in a specific orientation, and therefore should not be construed as a limitation of the present invention.

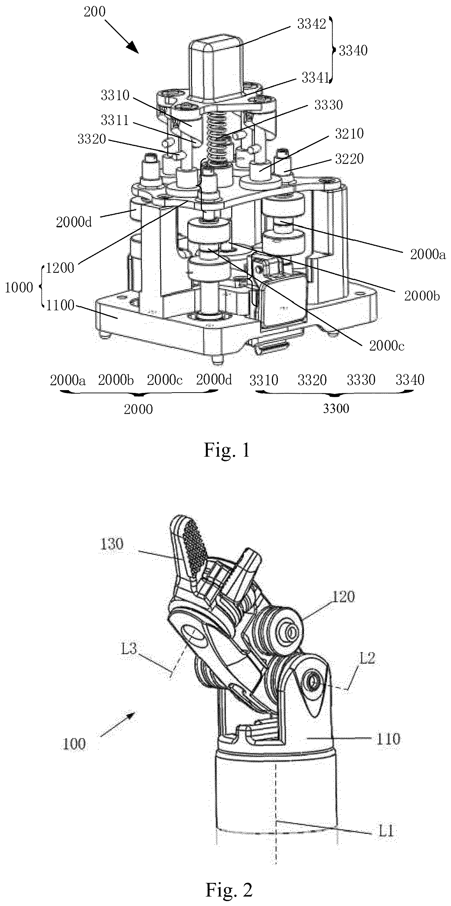

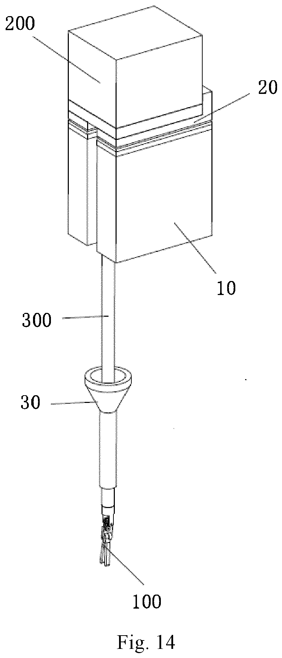

Please refer to Figs. 1 to 4 and 14, the surgical instrument according to the first embodiment of the present invention includes an end portion 100 and a case portion 200 including an instrument operating device therein (as shown in Fig. 14). The end portion 100 includes at least one joint defined with a zero position and a non-zero position. The instrument operating device includes a base 1000, joint drive shafts 2000 and a reset assembly. The base 1000 may include a first basal plate 1100 and a second basal plate 1200, which are disposed opposite to each other, and the second basal plate 1200 is located above the first basal plate 1100. The joint drive shafts 2000 are rotatably disposed on the first basal plate 1100 and are connected with the joints of the end portion 100 in a force transmittable manner. The reset assembly includes reset shafts 3100, transmission mechanisms 3200 and a reset mechanism 3300. The reset shafts 3100 are rotatably disposed on the second basal plate 1200 and are connected to the joint drive shafts 2000 by the transmission mechanisms 3200. When the joints of the end portion 100 are in the non-zero position, the reset mechanism 3300 is configured to drive the reset shafts 3100 to rotate, and then drive the joint drive shafts 2000 by means of the transmission mechanisms 3200, so as to drive the joints of the end portion 100 to move to the zero position. The zero position is considered to be a set position, which is used as a reference point for measuring other positions of the joints.

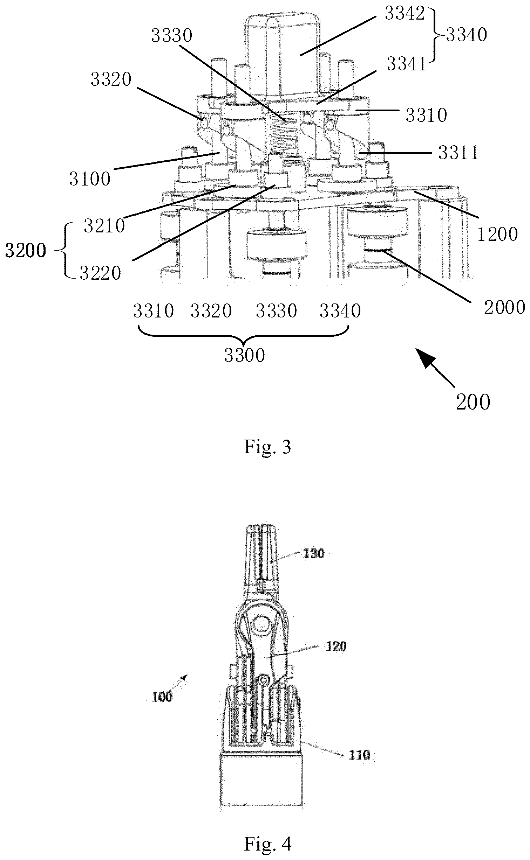

Please refer to Figs. 2 and 4, the end portion 100 may preferably include an end base 110, an actuator base 120, end actuators 130, an rotatory joint, a swing joint and two open/close joints. The end base 110 is rotatable around the first axis L1 by means of the rotatory joint. The actuator base 120 is arranged on the end base 110 and is rotatable around the second axis L2 by means of the swing joint. The end actuators 130 are disposed on the actuator base 120 and are both rotatable around the third axis L3 by means of one of the open/close joints. Preferably, the third axis L3 and the second axis L2 are perpendicular and do not intersect. In this embodiment, the specific type of the end actuator 130 is not particularly limited, and it can be selected according to actual needs. For example, the end actuator 130 may be an electric hook (in this case, only one open/close joint may be required) or scissors. In other embodiments, the end actuator 130 may be a flushing tube, and the end actuator 130 is fixedly arranged on the actuator base 120 in this case. For ease of understanding, in this embodiment, the end actuator 130 is implemented as scissors as an example for description. It should be understood that the number and the distribution of the joints of the end portion 100 are not limited to the above description. Those skilled in the art can set according to actual needs, which is not limited in this embodiment. In this embodiment, when the joints of the end portion 100 are in the zero position, the end portion 100 is farthest from the case portion 200, and the plane defined by the third axis L3 and the first axis L1 is coplanar with the symmetry plane of the first joint drive shaft 2000a with the second joint drive shaft 2000b.

Optionally, the end portion 100 further includes a serpentine joint (not shown in the figures) for enlarging the adjustment range of the end portion 100, and may include a plurality of members like snake bones. In this embodiment, the degrees of freedom of the serpentine joint and the shape of the serpentine are not particularly limited. For example, the serpentine joint includes two degrees of freedom, and the swing axis of the serpentine joint is preferably in parallel with the second axis L2 or the third axis L3.

Please refer to Fig. 1, in this embodiment, the number of the joint drive shafts 2000 is exemplarily four, which are a first joint drive shaft 2000a, a second joint drive shaft 2000b, a third joint drive shaft 2000c and a fourth joint drive shaft 2000d. The first joint drive shaft 2000a and the second joint drive shaft 2000b are respectively configured to drive the two blades of the scissors to rotate around the third axis L3 by meas of the open/close joint. The third drive shaft 2000c is configured to drive the actuator base 120 to rotate around the second axis L2 by measn of the swing joint. The fourth drive shaft 2000d is configured to drive the end base 110 to rotate around the first axis L1 by means of the rotatory joint. Correspondingly, the number of the reset shafts 3100 is also four. The four reset shafts 3100 are provided in a one-to-one correspondence with the four joint drive shafts 2000, and one of the reset shafts 3100 is connected with one of the joint drive shafts 2000 by one of the transmission mechanisms 3200.

Generally, each of the joint drive shafts 2000 has an end configured to connect with an external power mechanism and receive the torque generated by the external power mechanism, and a further end connected to the end portion 100 by a connecting structure to drive the corrsponding joint of the end portion 100 to move. In this embodiment, there is no particular limitation on the specific manner in which the joint drive shafts 2000 drive the end portion 100 by means of the connecting structure and the specific type of the connecting structure. The connecting structure can be a flexible member such as a wire rope, or a rigid member such as a combination of a transmission rod and a gear.

Preferably, the surgical instrument further includes an instrument shaft 300 (as shown in Fig. 14). Two ends of the instrument shaft 300 are respectively connected to the end portion 100 and the case portion 200, and the axis of the instrument shaft 300 may be collinear with the first axis L1. The connecting structure extends to the end portion 100 through the instrument shaft 300, so as to avoid external interference.

Please continue to refer to Fig. 1, the second basal plate 1200 is fixedly arranged above the first basal plate 1100 by a support member. The second basal plate 1200 is defined with through holes (not annotated in the figure) allowing the joint drive shafts 2000 to pass therethrough. The lower end of each of the joint drive shafts 2000 is rotatably disposed on the first basal plate 1100, and the upper end of each of the joint drive shafts 2000 extends upward through the through hole to a location above the second basal plate 1200. Preferably, first bearings (not shown in the figure) are provided at the through holes, and the joint drive shafts 2000 are connected to the second basal plate 1200 through the first bearings to avoid radial runouts of the the joint drive shafts 2000 during their rotations driven by an external power mechanism, which may affects the motion accuracy of the end portion 100. The reset shafts 3100 are rotatably disposed on the second basal plate 1200. Preferably, the reset shafts 3100 are arranged in parallel with the joint drive shafts 2000.

The transmission mechanisms 3200 are disposed above the second basal plate 1200, and preferably the transmission mechanisms 3200 are deceleration transmission mechanisms, and the transmission ratio of each of the transmission mechanisms 3200 can be set to n: 1, where n is a natural number greater than 1. In this way, the rotation angle ratio between each reset shaft 3100 and the correspoding one of the joint drive shafts 2000 is 1: n. Generally, in order to precisely control the rotation angle of the joint, the transmission ratio between each of the joint drive shafts 2000 and the corresponding joint is set to 1. Therefore, if the unidirectional rotation angle of the joint of the end portion 100 is greater than the unidirectional rotation angle of the corresponding reset shaft, the corresponding n value is selected according to the maximum angle of unidirectional rotation of the joint and the maximum angle of unidirectional rotation of the reset shaft 3100, so that the joint at the end portion 100 can be reset from a non-zero position at any angle to the zero position. For example, the maximum unidirectional rotation angle of the reset shaft 3100 is 180°, and the unidirectional rotation angle of the joint is 360°. By designing the transmission ratio of the transmission mechanism 3200 to be 2:1, it can be achieved that the joint moves from a non-zero position deviated by any angle to the zero position. According to actual needs, the transmission ratio of the transmission mechanism 3200 may be in the range of 1:1-5:1, that is, greater than 1: 1 and less than or equal to 5: 1.

Each of the transmission mechanisms 3200 may be an engagement transmission mechanism, such as a gear transmission mechanism, a chain transmission mechanism or a belt transmission mechanism, etc. In this embodiment, each of the transmission mechanisms 3200 is implemented as a gear transmission mechanism. Each of the transmission mechanism 3200 includes a driving gear 3210 and a driven gear 3220. The driving gear 3210 is configured to rotate synchronously with the reset shaft 3100, and the driven gear 3220 is configured to rotate synchronously with the corresponding one of the joint drive shafts 2000. Preferably, the driving gear 3210 is coaxially with and fixedly arranged on the reset shaft 3100, and the driven gear 3220 is coaxially with and fixedly arranged on the corresponding one of the joint drive shafts 2000. The driven gear 3220 is configured to engage with the driving gear 3210.

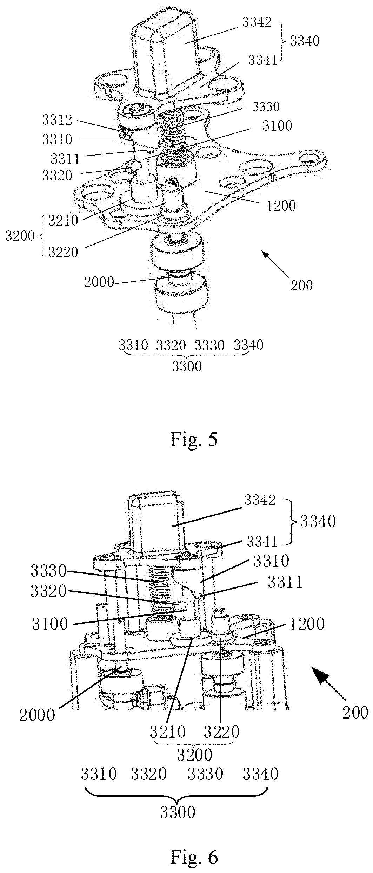

Referring to Figs. 1, 3, 5 and 6, the reset mechanism 3300 includes a drive member 3310, a force transmission member 3320 and an elastic support 3330. The force transmission member 3320 is connected with the reset shaft 3100, and the force transmission member 3320 and the reset shaft 3100 remain stationary relative to each other. The drive member 3310 is configured to drive the reset shaft 3100 to rotate by means of the force transmission member 3320, and the elastic support 3330 is configured to allow and disallow the force transmission between the force transmission member 3320 and the drive member 3310. The number of the drive members 3310 is not greater than the number of the reset shafts 3100. In this embodiment, the number of the drive members 3310 is four, and each of the drive members 3310 is connected with one of the reset shafts 3100, that is, all the four joints can be driven to the zero position.

The each of the drive members 3310 is sleeved on the corresponding reset shaft 3100, and the drive member 3310 is movable up and down along the axial direction of the reset shaft 3100, and remains stationary relative to the second basal plate 1200 in the circumferential direction. The cross section of the drive member 3310 is preferably circular. A surface of the drive member 3310 close to the force transmission member 3320 serves as a guide surface 3311. The guide surface 3311 is configured to guide the force transmission member 3320 to rotate around the axis of the reset shaft 3100. In this embodiment, the force transmission member 3320 is disposed on the reset shaft 3100 and is located below the drive member 3310. When the guide surface 3311 of the drive member 3310 is in contact with the force transmission member 3320, the force transmission member 3320 can move on the guide surface 3311, and the guide surface 3311 provides the force for rotating the force transmission member. Preferably, the force transmission member 3320 is implemented as a straight rod and is arranged perpendicular to the axis direction of the corresponding one of the joint drive shafts 2000, so as to achieve the movement of the force transmission member 3320 on the guide surface 3311. In addition, the force transmission member 3320 is also sleeved with ball bearings, so that the force transmission member 3320 can roll on the guide surface 3311. Optionally, the guide surface 3311 has a peak and a valley, for example, one peak and one valley, and the surface from the peak to the valley may be an inclined plane surface, so that the reset shaft 3100 can be rotated at a constant speed, or the surface from the peak to the valley is designed to be an inclined cruved surface, so that the reset shaft 3100 can be rotated at a variable speed.

In order to avoid excessive movement of the joint of the end portion 100 in the process during which the drive member 3310 is driven to rotate by the reset shaft 3100 and thus the corresponding one of the joint drive shafts 2000 is driven to rotate so that the joint of the end portion 100 returns to the zero position, the reset mechanism 3300 is further provided with a limiting mechanism. In an optional implementation, the limiting mechanism is a zero position slot 3312 defined in the guide surface 3311. The zero position slot 3312 is circumferentially sized to match with the force transmission member 3320, so that the zero position slot 3312 can accommodate the force transmission member 3320. The zero position slot 3312 is configured such that when the force transmission member 3320 rotates around the axis of the reset shaft 3100 under the action of the guide surface 3311 until the force transmission member 3320 enters the zero position slot 3312, the joint of the end portion 100 corresponding to the reset shaft 3100 just returns to the zero position. At this time, under the restriction of the zero position slot 3312, the reset shaft 3100 cannot rotate any more. Optionally, the zero position slot 3312 is disposed at the valley of the guide surface 3311.

In this embodiment, the number of the elastic support 3330 may be one. The elastic support 3330 is an elastic member, including but not limited to a spring. The reset mechanism 3300 further includes a pressing portion 3340, and the pressing portion 3340 includes a connection plate 3341 and a button 3342. The connection plate 3341 is disposed above the drive members 3310 and is connected to each of the drive members 3310. Since the pressing portion 3340 is connected with the plurality of the drive members 3310, the torque from the drive members 3310 can be eliminated, and thus the drive members 3310 can remain stationary in the circumferential directions thereof relative to the second basal plate 1200. Preferably, the connection plate 3341 is shaped to match with the positions where the four reset shafts 3100 are distributed. For example, the connection plate 3341 has an "X" shape. The button 3342 is disposed at the center of the upper surface of the connection plate 3341. The elastic support 3330 has upper and lower ends that are opposite with each other. The upper end is connected with or adjacent to the connection plate 3341, and the lower end is connected to the second basal plate 1200. Preferably, the elastic support 3330 are arranged in posional correspondence with the button 3342, that is, the four reset shafts 3100 are evenly arranged around one elastic support 3330.

Referring to Fig. 1, when the pressing portion 3340 is not subjected to an external force that causes the elastic suppor 3330 to deform, the elastic support 3330 supports the connection plate 3341 so that the drive members 3310 connected to the connection plate 3341 are apart from force transmission members 3320 without contacting each other (that is, there is no interaction force between the drive members 3310 and the force transmission members 3320). Please refer to Fig. 3, when the pressing portion 3340 receives an external force directing towards the second basal plate 1200, the elastic support 3330 is compressed, and each of the drive members 3310 faces the corresponding force transmitting member 3320. When the drive members 3310 moves until the force transmission member 3320 abuts against the guide surface 3311, the drive member 3310 starts to drive the force transmission member 3320 to rotate around the axis of the reset shaft 3100. The drive member 3310 continues to move under the action of the external force, and the force transmission member 3320 will move along the guide surface 3311 and rotate around the axis of the reset shaft 3100, so that the reset shaft 3100 rotates, and drives the corresponding one of the joint drive shafts 2000 to rotate by means of the transmission mechanism 3200, thereby driving the corresponding joint of the end portion 100 to move. It can be understood that the "external force" refers to the force coming from an element or mechanism other than the surgical instrument or an operator.

The following describes the process of performing minimally invasive surgery using the surgical instrument provided in this embodiment.

First, the surgical instrument and the external power mechanism are assembled. Usually, when the joint of the end portion 100 of the surgical instrument is in the zero position, it is beneficial to improve the success rate for the connection between the surgical instrument and the external power mechanism, and is more conducive to the insertion of the end portion 100 into the trocar 30 (please refer to Fig. 14). Therefore, before installing the surgical instrument, one shall visually determine whether the end portion 100 is in the zero position, if so, directly connect the surgical instrument to the external power mechanism; if not, press the button 3342 to move the drive member 3310 toward the force transmission member 3320 until the drive member 3310 contacts the force transmission member 3320. Under the action of the guide surface 3311, the reset shaft 3100 rotates, and the transmission mechanism 3200 transmits torque to the joint drive shafts 2000 to rotate the joint drive shafts 2000 and drive the joints of the end portion 100 to the zero position. The surgical instrument and the external power mechanism are then connected.

Next, the end portion 100 in the zero position is inserted into the trocar 30 and enters the body cavity in order for minimally invasive surgery. During the surgical operation, the operator drives the joint drive shafts 2000 to rotate by means of the external power mechanism to adjust each joint of the end portion 100. After the use of the end portion 100 is complete, each joint of the end portion 100 is generally in a non-zero position.

Next, the external power mechanism and the joint drive shafts 2000 are disconnected from each other.

Next, each joint of the end portion 100 is adjusted to be back to the zero position. The button 3342 is pressed to make the drive member 3310 to move toward the force transmission member 3320 until the drive member 3310 contacts the force transmission member 3320. The button 3342 is further pressed, during which, under the action of the guide surface 3311, the force transmission member 3320 rotates, and drives the reset shaft 3100 to rotate, thereby driving the joint drive shafts 2000 to rotate, thereby driving each joint of the end portion 100 to move to the zero point.

Finally, the end portion 100 is withdrawn from the trocar 30.

In this embodiment, all the drive members 3310 are connected to a same pressing portion 3340. The four reset shafts 3100 can be adjusted simultaneously by one pressing portion 3340, so that all the joints of the end portion 100 can return to the zero position.

It should be understood that in this embodiment, the surgical instrument has four joint drive shafts 2000 and four reset shafts 3100 as an example for description. In other embodiments, according to actual requirements, the numbers of the joint drive shafts 2000 and the reset shafts 3100 of the surgical instrument may be more than four or less than four, which is specifically determined according to the number of degrees of freedom of the end portion 100.

When the number of the reset shafts 3100 is two or more and the number of the elastic support 3330 is only one, the two or more reset shafts 3100 are preferably evenly arranged around the elastic support 3330. When the number of the reset shaft is only one and the number of the elastic supports is more than two, the two or more elastic supports are evenly arranged around the reset shaft, and the drive member is connected with the two or more of the elastic supports by the pressing portion, so that a force-balanced drive member is achieved (not shown).

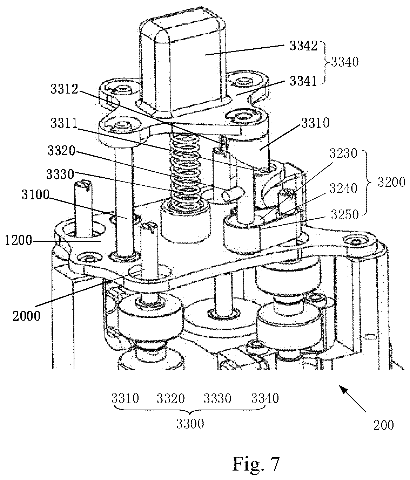

Fig. 7 is a schematic diagram showing a partial structure of a surgical instrument according to a second embodiment of the present invention. This embodiment differs from the first embodiment in that the transmission mechanism 3200 is a flat belt transmission mechanism. Specifically, the transmission mechanism 3200 includes a driving pulley 3230, a driven pulley 3240 and a transmission belt 3250. The driving pulley 3230 rotates synchronously with the reset shaft 3100, the driven pulley 3240 rotates synchronously with the the corresponding one of the joint drive shafts 2000, and the transmission belt 3250 is wound around the driving pulley 3230 and the driven pulley 3240. Preferably, the driving pulley 3230 is coaxially fixed to the corresponding reset shaft 3100, and the driven pulley 3240 is coaxially fixed to the corresponding one of the joint drive shafts 2000. According to actual needs, any of the technology of open belt drive, cross belt drive, and quarter-twist belt drive can be applied to the transmission mechanism 3200.

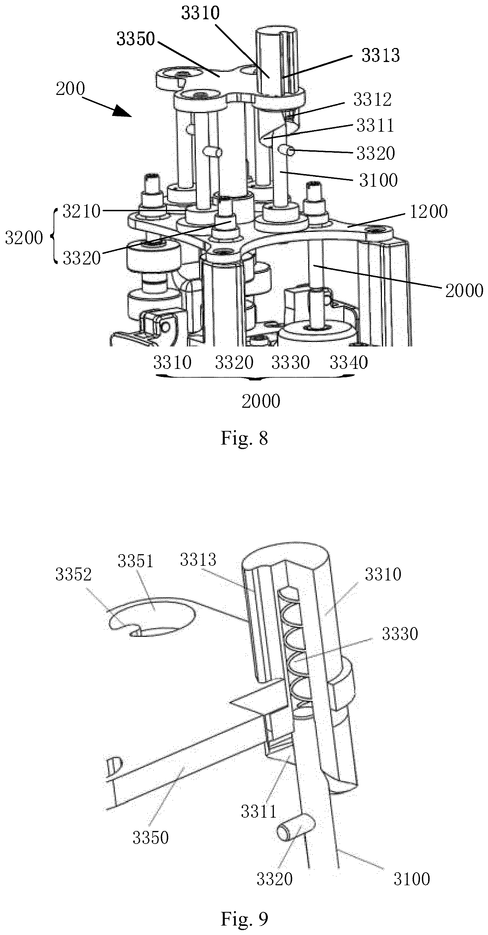

Fig. 8 is a schematic diagram showing a partial structure of a surgical instrument according to a third embodiment of the present invention, and Fig. 9 is a partial cross-sectional view of the surgical instrument shown in Fig. 8. As shown in Figs. 8 and 9, in this embodiment, the operator can drive only one reset shaft 3100 to rotate at a time, so that one of the joint drive shafts 2000 corresponding to the reset shaft 3100 can be drived to rotate, and the joint controlled by the corresponding one of the joint drive shafts 2000 can return to the zero position as shown. As a result, the reset operation is more flexible.

Please refer to Figs. 8 and 9, this embodiment differs from the first embodiment in that each of the drive members 3310 is provided with a blind mounting hole, and is sleeved on the corresponding reset shaft 3100 as shown. The elastic support 3330 is disposed in the blind mounting hole, and supports the drive member 3310 in the axial direction of the reset shaft 3100.

Optionally, as shown in Figs. 8 and 9, in the axial direction of each of the reset shafts 3100, the elastic support 3330 has first and second ends that are opposite with each other (taking the orientation as shown as an example, the first end is the upper end, and the second end is the lower end). The second end of the elastic support 3330 abuts against the upper end surface of the reset shaft 3100, and the first end of the elastic support 3330 abuts against the top wall of the blind mounting hole. In this way, when the operator presses the drive member 3310, the drive member 3310 moves downward along the axial direction of the reset shaft 3100, so that the guide surface 3311 of the drive member 3310 contacts the corresponding force transmission member 3320, thereby driving the reset shaft 3100 to rotate.

Further, please refer to Fig. 9, in order to prevent the drive member 3310 from skewing during the downward movement, the reset mechanism 3300 further includes a seat 3350 located above the second basal plate 1200, and the plane where the seat 3350 is located is perpendicular to the axis of the reset shaft 3100. The seat 3350 is defined with a through hole 3351 having a diameter matching with the outer diameter of the drive member 3310, so that the drive member 3310 can be guided to move up and down in the through hole 3351. Further, a protrusion 3352 is provided on the circumference of the through hole 3351, and a groove 3313 extending along the axial direction of the drive member 3310 is defined on the drive member 3310. The protrusion 3352 is matched with the groove 3313 to keep the drive member 3310 stationary in the circumferential direction thereof relative to the second basal plate 1200. Preferably, in the circumferential direction of the drive member 3310, the groove 3313 is aligned with the zero position slot 3312.

In an alternative embodiment, a second bearing (not shown in the figures) is provided at the blind mounting hole, the outer ring of the second bearing is fixed to the wall of the blind mounting hole, and the reset shaft 3100 is slidably connected with the inner ring of the second bearing. The first end of the elastic support 3330 is in contact with the top wall of the blind mounting hole, and the second end of the elastic support 3330 is connected with the upper end surface of the outer ring of the second bearing. The advantage of this arrangement is that when the drive member 3310 drives the reset shaft 3100 to rotate, the elastic support 3330 will not rotate together with the reset shaft 3100, thereby avoiding the torsion of the elastic support 3330.

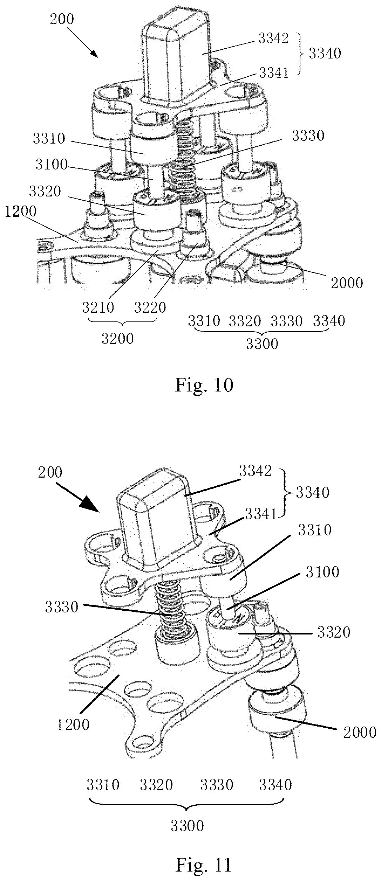

Figs. 10 to 12 are schematic diagrams showing a partial structure of a surgical instrument according to a fourth embodiment of the present invention. As shown in Figs. 10 to 12, this embodiment differs from the first embodiment in that the drive members 3310 and the force transmission members 3320 are made of magnets.

Specifically, each of the drive members 3310 includes a first magnet, and each of the force transmission member 3320 includes a second magnet. The first magnet is connected to the elastic support 3330 by the pressing portion 3340, and the first magnet is sleeved on the corresponding reset shaft 3100 and can move along the axial direction of the reset shaft 3100, while the first magnet remains stationary relative to the second basal plate 1200 in the circumferential direction. The second magnet is fixedly arranged on the reset shaft 3100.

When the elastic support 3330 is not deformed by an external force, the elastic support 3330 supports the first magnet, so that the first magnet is apart from and the second magnet with a predetermined distance ensuring that no magnetic force is generated between the first magnet and the second magnet. When the elastic support 3330 is compressed under the action of an external force, the first magnet moves toward the second magnet to reduce the distance between the first magnet and the second magnet. When the distance between the first magnet and the second magnet is reduced to a certain extent, a magnetic force is generated between the first magnet and the second magnet, and the magnetic force will drive the reset shaft 3100 to rotate, and then drive the corresponding one of the joint drive shafts 2000 to rotate, so as to make the joint of the end portion 100 move to the zero position.

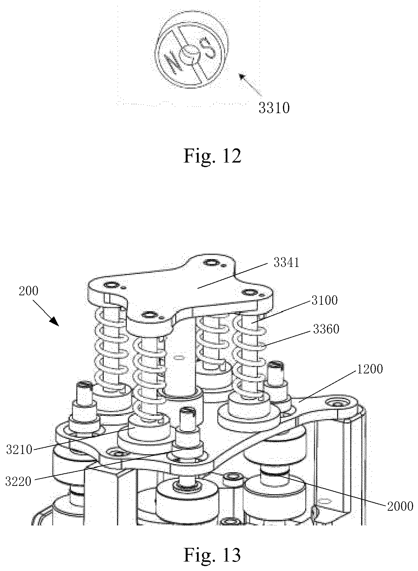

Optionally, in this embodiment, each of the first magnet and the second magnet has a cylindrical structure. As shown in Fig. 12, the first magnet has a first surface (that is the surface close to the second magnet), and the first surface is provided with one N pole and one S pole, which are arranged around the axis of the first magnet (preferably parallel to the axis of the reset shaft 3100). The structure of the second magnet may be the same as that of the first magnet, that is, the second magnet has a second surface (that is a surface opposite to the first surface of the first magnet), and the second surface is provided with one N pole and one S pole, which are arranged around the axis of the second magnet (preferably parallel to the axis of the reset shaft 3100). When the joint of the end portion 100 is in the zero position, the N pole of the first surface and the S pole of the second surface are disposed directly opposite with each other, and the S pole of the first surface and the N pole of the second surface are disposed directly opposite with each other. In this way, when the joint of the end portion 100 is in a non-zero position, the N pole of the first surface is at least partially opposite to the N pole of the second surface, and the S pole of the first surface is at least partially opposite to the S pole of the second surface. At this time, the operator presses the pressing portion 3340 to move the first magnet toward the second magnet, and then a repulsive magnetic force is generated between the first magnet and the second magnet, and under the action of the magnetic force, the reset shaft 3100 rotates to make the first magnet and the second magnet return to the positions where the S pole of the first surface and the N pole of the second surface are opposite with each other, and the N pole of the first surface and the S pole of the second surface are opposite with each other. During this process, the reset shaft 3100 drives the corresponding one of the joint drive shafts 2000 to rotate by means of the transmission mechanism 3200, so as to drive the joint of the end portion 100 to return to the zero position.

In this embodiment, the magnetic force drives the reset shaft 3100 to rotate at an angle which can be 180° at maximum in one direction. When the maximum unidirectional rotation angle of the joint of the end portion 100 is greater than 180°, the transmission ratio of the transmission mechanism 3200 is set so that the joint can return to the zero position from a non-zero position deviating from the zero position at any angle.

In addition, after the joint of the end portion 100 returns to the zero position, the operator no longer presses the pressing portion 3340, and the elastic support 3330 returns to the original shape, so that the first magnet is apart from the second magnet, during which since the first magnet and the second magnet attract each other, the change of the magnetic force between the two magnets due to the increase in the distance will not cause the reset shaft 3100 to rotate, so that the position of the joint of the end portion 100 will not be affected.

It can be understood that the numbers of N poles and S poles on the first surface of the first magnet and the second surface of the second magnet in this embodiment can be determined according to actual needs. For example, the numbers of N poles and S poles on the first surface may be both two, and the N poles and the S poles are alternately arranged around the axis of the first magnet. Similarly, two N poles and two S poles may be disposed on the second surface of the second magnet, and the N poles and the S poles are alternately arranged around the axis of the second magnet.

Fig. 13 is a schematic diagram showing a partial structure of a surgical instrument according to a fifth embodiment of the present invention. As shown in Fig. 13, in this embodiment, the reset mechanism 3300 includes torsion springs 3360 each sleeved on the corresponding reset shaft 3100. In the axial direction of the reset shaft 3100, the torsion spring has a third end and a fourth end, which are opposite to each other (taking the orientation as shown as an example, the third end is the upper end, the fourth end is the lower end), the third end of the torsion spring 3360 is configured to remain stationary in the circumferential direction thereof relative to the second basal plate 1200, and the fourth end of the torsion spring 3360 can rotate synchronously with the reset shaft 3100. Specifically, the reset mechanism 3300 further includes a connection plate 3341 that can remain stationary relative to the second basal plate 1200 (e.g., the connection plate 3341 is fixed to the second basal plate 1200) to eliminate the torque from the torsion spring 3360. The upper end of the torsion spring 3360 is connected to the connection plate 3341, and the lower end of the torsion spring 3360 is connected to the reset shaft 3100, so that the lower end of the torsion spring 3360 rotates synchronously with the reset shaft 3100. The torsion spring 3360 is configured such that when the joint of the end portion 100 is in the zero position, the torsion spring 3360 is not elastically deformed, and when the joint of the end portion 100 is in the non-zero position, the torsion spring 3360 is deformed to store elastic potential energy.

When the surgical instrument provided in this embodiment is used for minimally invasive surgery, the joint drive shafts 2000 are connected to an external power mechanism, and the joint drive shafts 2000 are driven by the external power mechanism to rotate along a first direction to drive the repsective joints of the end portion 100 to move, so that the joints of the end portion 100 are in non-zero positions, and the posture of the end portion 100 is adjusted. During this process, each of the joint drive shafts 2000 drives the corresponding reset shaft 3100 to rotate by means of the corresponding transmission mechanism 3200, and the lower end of the torsion spring 3360 rotates synchronously with the reset shaft 3100 and deforms to store elastic potential energy. When the use of the end portion 100 is complete, the external power mechanism is disconnected from the joint drive shafts 2000, and each of the torsion springs 3360 releases elastic potential energy accordingly to drive the corresponding reset shaft 3100 to rotate, and the reset shaft 3100 drives the corresponding one of the joint drive shafts 2000 to rotate in a second direction by means of the correspoding transmission mechanism 3200, so as to drive the end portion 100 to return to the zero position. The second direction is opposite to the first direction. When the first direction is a counterclockwise direction, the second direction is a clockwise direction, and vice versa. The advantage of this embodiment is that the joint of the end portion 100 can be automatically returned to the zero position without the operator exerting additional force on the surgical instrument.

It should be understood that when the surgical instrument is used during surgery, the current posture of the end portion 100 needs to be maintained, and at this time, the external power mechanism is still connected to the joint drive shafts 2000 and exerts a force on the joint drive shafts 2000 to keep the joint drive shafts 2000 relatively stationary. That is to say, in this embodiment, the elastic potential energy stored by each torsion spring 3360 is insufficient to overcome the force exerted on the corresponding one of the joint drive shafts 2000 from the external power mechanism, and only after the external power mechanism is disconnected from the joint drive shafts 2000, the torsion springs 3360 can drive the reset shafts 3100 to rotate.

Referring to Fig. 14, an embodiment of the present invention also provides a surgical instrument system including a power case 10 and the aforementioned surgical instrument. The joint drive shafts (not shown in Fig. 14) are configured to connect with the power case 10 and rotate under the drive of the power case 10. That is, the power case 10 includes the aforementioned external power mechanism.

The surgical instrument system further includes a sterile accessory 20 that is disposed between the power case 10 and the case portion 200, and configured to detachably connect the power case 10 and the case portion 200 respectively. The torque provided by the power case 10 is transmitted to the joint drive shafts 2000 of the case portion 200 through the sterile assembly 20, thereby driving the joints of the end portion 100 to move.

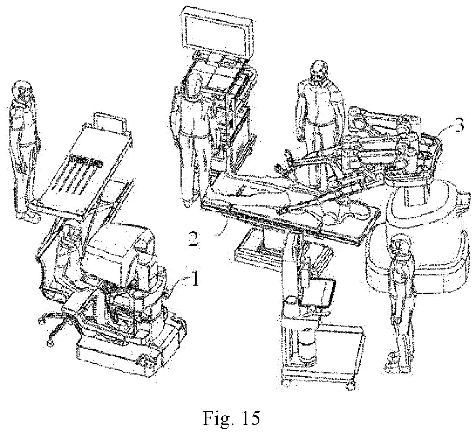

An embodiment of the present invention also provides a surgical robot including the aforementioned surgical instrument system. Specifically, please refer to Figs. 14 and 15, the surgical robot includes a control station and an actuating station. The control station is provided with a console 1 with a master manipulator, and the actuating station includes an surgical cart 2 and a side cart 3, etc. The surgical cart 2 is configured to allow a patient to lie thereon for surgery. The side cart 3 accommodates a robotic arm (not annotated in the figures) for mounting the surgical instrument system. The power case 10 is fixed to the end of the robotic arm. The surgical instrument of the surgical instrument system is mounted to the robotic arm and is detachably connected to the power case 10. Mapping relationships between the robotic arm, the surgical instrument system and the master manipulator are predetermined, so that they form a master-slave relationship, and the robotic arm and the surgical instrument system can move in various directions based on the corresponding movements of the master manipulator to complete the surgical operation.

Although the present invention is disclosed above, it is not limited thereto. It is apparent that those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A surgical instrument, comprising an end portion and an instrument operating device, the end portion comprising at least one joint defined with a zero position and a non-zero position; the instrument operating device comprising a joint drive shaft and a reset assembly, and the joint drive shaft connected with the joint of the end portion in a force transmitable manner; the reset assembly comprising a reset shaft, a transmission mechanism and a reset mechanism, and the reset shaft connected with the joint drive shaft by the transmission mechanism, and wherein
when the joint of the end portion is in the non-zero position, the reset mechanism is configured to drive the reset shaft to rotate, to drive the joint drive shaft to rotate through the transmission mechanism, so as to drive the corresponding joint of the end portion to move to the zero position.

2. The surgical instrument according to claim 1, further comprising an case portion in which the instrument operating device is arranged.

3. The surgical instrument according to claim 1, wherein the transmission mechanism is a deceleration transmission mechanism.

4. The surgical instrument according to claim 1, wherein the instrument operating device comprises a first basal plate and a second basal plate that are arranged opposite to each other, the first basal plate and the second basal plate spaced apart from each other and remaining stationary relative to each other, the joint drive shaft rotatably disposed on the first basal plate and extending through the second basal plate, the reset shaft rotatably disposed on the second basal plate, and connected with a portion of the joint drive shaft extending out of the second basal plate by the transmission mechanism.

5. The surgical instrument according to claim 3, wherein a reduction ratio of the transmission mechanism ranges from 1:1 to 5: 1.

6. The surgical instrument according to claim 5, wherein the transmission mechanism is an engagement transmission mechanism.

7. The surgical instrument according to claim 6, wherein the transmission mechanism is a gear transmission mechanism comprising a driving gear and a driven gear, the driving gear configured to rotate synchronously with the reset shaft, and the driven gear configured to rotate synchronously with the joint drive shaft, and the driven gear engaged with the driving gear.

8. The surgical instrument according to claim 5, wherein the transmission mechanism is a flat belt transmission mechanism comprising a driving pulley, a driven pulley and a transmission belt, the driving pulley configured to rotate synchronously with the reset shaft, the driven pulley configured to rotate synchronously with the joint drive shaft, and the transmission belt is wound around the driving pulley and the driven pulley.

9. The surgical instrument according to claim 4, wherein the reset mechanism comprises a drive member, a force transmission member and an elastic support, and the force transmission member disposed on the reset shaft and remaining stationary relative to the reset shaft, the drive member configured to drive the reset shaft to rotate through the force transmission member, and the elastic support configured to allow and disallow a force transmission between the reset shaft and the drive member.

10. The surgical instrument according to claim 9, wherein the drive member is sleeved on the reset shaft, and remains stationary in a circumferential direction thereof relative to the second basal plate, and an end face of the drive member facing the force transmission member is formed as a guide surface; wherein
the drive member, the force transmission member and the elastic support are configured such that when the elastic support is not subjected to an external force causing a deformation thereof, the elastic support is configured to support the drive member so that the drive member is apart from the force transmission member; when the joint of the end portion is in the non-zero position and the elastic support is compressed under an action of an external force, the drive member moves toward the force transmission member to cause the guide surface to guide the force transmission member to rotate around an axis of the reset shaft so that the reset shaft is drived to rotate to move the corresponding joint of the end portion to the zero position.

11. The surgical instrument according to claim 10, wherein when the drive member is in contact with the force transmission member, the force transmission member is movable on the guide surface, and the guide surface provides a force for rotating the force transmission member to drive the force transmission member to rotate around the axis of the reset shaft.

12. The surgical instrument according to claim 11, further comprising a limit mechanism configured to prevent the reset shaft from rotating when the corresponding joint of the end portion moves to the the zero position.

13. The surgical instrument according to claim 12, wherein the limiting mechanism is a zero position slot defined in the guide surface, and the zero position slot is circumferentially sized to match with force transmission member; when the joint of the end portion returns to the zero position, the force transmission member is accommodated in the zero position slot.

14. The surgical instrument according to claim 13, wherein the guide surface has a peak and a valley, and the zero position slot is arranged at the valley.

15. The surgical instrument according to claim 9, wherein the drive member comprises a first magnet, and the force transmission member comprises a second magnet, and wherein the first magnet is connected to the elastic support, and the first magnet is sleeved on the reset shaft and remains stationary in a circumferential direction thereof relative to the second basal plate, and the first magnet is movable along an axial direction of the reset shaft, and the second magnet is fixedly arranged on the reset shaft; wherein
the drive member and the elastic support are configured such that when the elastic support is not subjected to an external force causing a deformation thereof, no magnetic force is generated between the first magnet and the second magnet; when the joint of the end portion is in the non-zero position and the elastic support is compressed and deformed under an action of an external force, the first magnet moves toward the second magnet, so that a magnetic force is generated between the first magnet and the second magnet to drive the reset shaft to rotate for causing the corresponding joint of the end portion to move to the zero position.

16. The surgical instrument of claim 15, wherein the first magnet has a first surface and the second magnet has a second surface, and the first surface faces the second surface, and wherein at least one N pole and at least one S pole are arranged on the first surface, and at least one N pole and at least one S pole are arranged on the second surface, when the joint of the end portion is in the zero position, the N pole on the first surface is arranged directly opposite to the S pole on the second surface, and the S pole on the first surface is arranged directly opposite to the N pole on the second surface.

17. The surgical instrument according to any one of claims 9 to 16, wherein the reset mechanism further comprises a pressing portion configured to allow the drive member to be connected to the elastic support, and keep the drive member stationary in a circumferential direction thereof relative to the second basal plate.

18. The surgical instrument according to claim 17, wherein a number of the elastic support is one, a number of the reset shafts is at least two, and the at least two drive shafts are evenly arranged around the one elastic support.

19. The surgical instrument according to claim 10, wherein a blind mounting hole is formed on the drive member and sleeved on the reset shaft, and the elastic support is disposed in the blind mounting hole; the elastic support has a first end and a second end that are opposite to each other along an axial direction of the reset shaft, the first end abutting on a top wall of the blind mounting hole, and the second end abutting against an end face of the reset shaft.

20. The surgical instrument according to claim 10, wherein a blind mounting hole is formed on the drive member, a bearing is provided at the blind mounting hole, and the reset shaft is configured to be inserted into the blind mounting hole and connected with the drive member by the bearing; the elastic support is arranged in the blind mounting hole, and the elastic support has a first end and a second end that are opposite to each other along an axial direction of the reset shaft, the first end abutting on a top wall of the blind mounting hole, and the second end fixedly connected with the bearing.

21. The surgical instrument according to claim 19 or 20, wherein the reset mechanism further comprises a seat defined therein with a through hole, the seat perpendicular to the reset shaft and arranged at a side of the second basal plate away from the first basal plate, the seat remaining stationary relative to the second basal plate, and the drive member configured to move in the through hole along the axial direction of the reset shaft.

22. The surgical instrument according to claim 21, wherein a protrusion is provided at the through hole, the drive member is defined thereon with a groove extending along an axial direction of the drive member, and the protrusion is matched with the groove, so that the drive member remains stationary in the circumferential direction relative to the second basal plate.

23. The surgical instrument according to claim 22, wherein a zero position slot is formed in the guide surface, and the zero position slot is circumferentially sized to match with the force transmission member; when the joint of the end portion returns to the zero position, the force transmission member is accommodated in the zero position slot, and the zero position slot is aligned with the groove in the circumferential direction of the drive member.

24. The surgical instrument according to claim 4, wherein the reset mechanism comprises a torsion spring that is sleeved on the reset shaft and has a third end and a fourth end that are opposite to each other, the third end configured to remain stationary in a circumferential direction thereof relative to the second basal plate, the fourth end connected with the reset shaft, and the fourth end being rotatable synchronously with the reset shaft;
the torsion spring is configured to store elastic potential energy when the joint of the end portion is in the non-zero position, and to drive the reset shaft to rotate and drive the joint drive shaft to rotate when the torsion spring releases the elastic potential energy, so as to move the joint of the end portion to the zero position.

25. The surgical instrument according to claim 24, wherein the reset mechanism comprises a connection plate connected with the third end of the torsion spring, so that the third end remains stationary in a circumferential direction thereof relative to the second basal plate.

26. A surgical instrument system, comprising a power case and the surgical instrument according to any one of claims 1-25, wherein the joint drive shaft is configured to receive power output from the power case and drive the joint to move.

27. A surgical robot, comprising the surgical instrument system according to claim 26 and a robotic arm configured to connect with the surgical instrument system.
